# FASCICULE DE BREVET EUROPEEN

(11) **EP 2 739 166 B1**
(45) Date de publication et mention de la délivrance du brevet: **26.02.2020**
(21) Numéro de dépôt: 12740973.8
(22) Date de dépôt: 30.07.2012
(51) Int. Cl.: A23L 33/19, A23L 33/185, A23L 2/66, A23C 9/20

(54) **MÉLANGE PROTÉIQUE FONCTIONNEL ET STABLE POUR COMPOSITIONS ALIMENTAIRES DESTINÉES AUX PERSONNES NÉCESSITANT UNE AMÉLIORATION ET UN MAINTIEN DE LEUR CONDITION PHYSIQUE.**
FUNKTIONELLE UND STABILE PROTEINMISCHUNG FÜR LEBENSMITTEL FÜR MENSCHEN, DIE IHRE KÖRPERLEISTUNG VERBESSERN ODER BEHALTEN BRAUCHEN
FUNCTIONAL AND STABLE PROTEIN MIXTURE FOR FOOD COMPOSITIONS FOR PERSONS IN NEED OF AN IMPROVEMENT OR THE MAINTENANCE OF THEIR BODY CONDITION

(30) Priorité: 01.08.2011 FR 1157052
(43) Date de publication de la demande: 11.06.2014
(73) Titulaire: Groupe Lactalis, 53000 Laval (FR)
(72) Inventeur: MORGAN, François, F-53000 Laval (FR); BONHOMME, Cécile, F-53000 Laval (FR); MATHIEU, Géraldine, F-53000 Laval (FR); LE RUYET, Pascale, F-53000 Laval (FR); BIGOT, Jean-Jacques, F-53000 Laval (FR)
(74) Mandataire: Cabinet Le Guen Maillet
(86) Numéro de dépôt international: PCT/EP2012/064846
(87) Numéro de publication internationale: WO 2013/017553

(56) Documents cités:
- WO-A1-2009/113858
- WO-A1-2010/126353
- FR-A1- 2 882 896
- US-A1- 2005 136 170
- US-A1- 2005 152 887
- JACKMAN RL, YADA RY: "Functional properties of whey-pea protein composite blends in a model system", J. FOOD SCI., vol. 54, no. 5, 1989, pages 1287-1292, XP002667766,
- SEEMA PATEL: "Functional food relevance of whey protein: A review of recent findings and scopes ahead", JOURNAL OF FUNCTIONAL FOODS, vol. 19, 1 December 2015 (2015-12-01), pages 308-319, XP055411335, NL ISSN: 1756-4646, DOI: 10.1016/j.jff.2015.09.040

## Description

La présente invention concerne une composition alimentaire liquide contenant un mélange protéique pour l'alimentation des personnes adultes, des sportifs, des personnes âgées, ou des personnes nécessitant une amélioration de leur condition physique, telles que des personnes malades, alitées, en état de faiblesse , en dénutrition ou soufrant de sarcopénie.

Les fonctions physiques dépendent d'une forte interaction entre le statut nutritionnel, l'homéostasie métabolique et le fonctionnement physiologique des tissus. L'âge et la malnutrition sont associés aux troubles métaboliques ainsi qu'aux disfonctionnements physiologiques. Cela aboutit à un déclin de la masse musculaire, de la masse osseuse ainsi qu'à des troubles cognitifs.

Chez les personnes âgées, les troubles nutritionnels sont fréquents et résultent par exemple d'isolement, de dépression, de maladies, de handicaps ou encore de troubles de la sensation du goût. Les carences peuvent encore être le résultat d'une diminution de l'anabolisme liée à l'âge. Des études ont montré que l'alimentation de la personne âgée devait être adaptée. Les carences en éléments nutritifs sont responsables du déclin de l'état de santé général de la personne âgée et notamment du déclin de la masse musculaire, de la masse osseuse et des fonctions cognitives.

La sarcopénie, par exemple, est un syndrome caractérisé par une perte progressive et généralisée de la masse et de la force ou de la performance du muscle squelettique entraînant des risques de complications tels qu'une incapacité physique, une pauvre qualité de vie, une faiblesse générale, susceptibles de provoquer même le décès de la personne. Cette diminution de la masse musculaire est le résultat, par exemple, d'une baisse, voire d'une absence d'activité physique, mais également d'une nutrition non adaptée et d'une baisse de la sensibilité du tissu musculaire aux apports nutritionnels. En effet, le muscle squelettique est un réservoir de protéines et d'acides aminés utilisés comme substrats énergétique et impliqués dans la synthèse des protéines responsables des mécanismes de défense. La sarcopénie est donc un problème majeur de santé publique.

Au sein des tissus de l'organisme ont lieu alternativement deux phénomènes : la synthèse et la dégradation des protéines. La quantité de protéines d'un tissu dépend donc de l'équilibre résultant de ces deux voies métaboliques. La synthèse des protéines dépend de nombreux facteurs dont la prise alimentaire. Suite à une prise alimentaire, on observe ainsi un gain de protéines associé à une augmentation de la synthèse des protéines et à une diminution de leur catabolisme. Les acides aminés apportés par l'alimentation sont en partie responsables de la protéosynthèse. Chez la personne souffrant de sarcopénie, l'une de ses voies, ou les deux, se trouve altérée.

L'augmentation de l'apport protéique ou le choix qualitatif de la ou des protéines apportées, associée, quand cela est possible, à de l'exercice physique est donc un facteur important dans la prévention et la lutte contre l'affaiblissement général de la personne âgée ou se trouvant en état de dénutrition. La quantité et la qualité des acides aminés apportés par cette supplémentation dans l'alimentation sont essentielles. Par exemple, des études indiquent que la supplémentation en protéines d'origine laitière, riches en leucine, est une voie possible pour prévenir la sarcopénie. A ce titre, les protéines de lactosérum se sont avérées efficaces dans la stimulation de la synthèse musculaire (Farnfield et al., 2005). En effet, la composition en acides aminés des protéines de lactosérum est proche de celle des protéines du muscle squelettique humain. Elles sont également riches en acides aminés branchés à chaîne ramifiée, tels que la leucine, qui sont métabolisés directement dans le tissu musculaire.

Un inconvénient lié à l'utilisation des protéines de lactosérum dans des compositions alimentaires est qu'elles sont instables à haute température et rapidement dénaturées, notamment à des températures comprises entre 70 et 90°C. Malheureusement, la préparation des compositions destinées à l'alimentation implique souvent un traitement de stérilisation par la chaleur, au cours duquel les températures utilisées vont bien au delà encore des températures de dénaturation des protéines. De telles protéines ne sont plus stables ce qui aboutit à des phénomènes de sédimentation visible et d'augmentation de la viscosité du produit. Outre l'aspect qualitatif dégradé du produit, les protéines précipitées perdent de leur efficacité fonctionnelle et peuvent également engendrer des réactions d'allergie.

Un des buts de la présente invention est de proposer une composition alimentaire liquide incorporant un mélange de protéines dans laquelle le mélange demeure fonctionnel et stable dans le temps, notamment après avoir été soumis à des traitements par hautes températures, qui ne sédimente pas et qui n'altère pas la viscosité des compositions dans lesquelles il est incorporé.

Un autre but de la présente invention est de proposer une composition alimentaire, incorporant un tel mélange, qui présente des qualités nutritionnelles et fonctionnelles et qui conserve ses qualités dans le temps, même après avoir subit un traitement thermique de stérilisation, sans que soient observés de phénomènes de sédimentation.

Encore un autre but de la présente invention est de proposer une telle composition alimentaire liquide pour son utilisation dans l'alimentation de la personne adulte, sportive, âgée ou malade, faible ou en situation de dénutrition ou encore risquant de l'être, et le maintien ou l'amélioration de sa condition physique et de son état de santé général.

A cet effet, l'invention concerne une composition alimentaire liquide avant subi un traitement thermique de stérilisation comprenant entre 2g et 20g pour 100 ml d'un mélange protéique constitué d'un concentré, ou isolât, de protéines solubles de lait pour 75 à 85 % et de protéines de pois pour 15 à 25 %, en poids par rapport au poids total du mélange de protéines.

Il a été en effet découvert que la combinaison des protéines de pois et des protéines solubles de lait permettait aux protéines solubles de conserver leurs propriétés fonctionnelles, c'est à dire nutritionnelles, et au mélange de demeurer stable, sans phénomène de sédimentation, après traitement à haute température et longue conservation, par exemple lorsqu'il est incorporé dans une composition subissant un tel traitement.

La demande de brevet WO2010/126353 décrit des compositions nutritionnelles liquides en particulier pour nutrition par sonde et contenant des mélanges de protéines contenant plus de 25% de protéines de pois et au plus 75% de protéines d'origine laitière. Les protéines de pois sont ici utilisées afin de réduire les couts de production plus faibles que lorsque des protéines d'origine laitière sont utilisées en tant qu'unique source.

Le brevet FR 2 882 896 décrit une composition alimentaire pour prévenir le syndrome de fragilité chez les personnes âgées, incluant la sarcopénie. La composition comprend des protéines naturelles riches en acides aminés à chaînes ramifiées, telles que des protéines de lactosérum et des protéines de pois selon un rapport de 62% de protéines de lactosérum et 37 %, environ, de protéines de pois.

La publication « »Functional properties of whey-pea protein composite blends in a model system », R. L. Jackman et R. Y. Yada, Journal of food science, 1989, Vol. 54, No5, est une publication scientifique dans laquelle il est expliqué que les protéines de pois sont intéressantes en tant qu'ingrédient alimentaire et, en particulier de protéines diététiques mais que, néanmoins, elles présentent de pauvres propriétés fonctionnelles d'où l'idée de les mélanger à des protéines laitières. Au cours de l'étude, ont été testés différents ratios de protéines de lactosérum/protéines de pois. Le but était de trouver le ratio pour lequel on observait de meilleurs qualités fonctionnelles par rapport à des protéines d'origine 100% de pois. Des tests de thermo coagulation sont décrits dans cet article. Ces tests consistent à chauffer le mélange à une T° de 80°C durant 15 secondes ce qui est éloigné des traitements hautes températures de type stérilisation UHT (140°C à 150°C).
Au sens de l'invention, on entend par « concentré ou isolat de protéines solubles de lait », un concentré de protéines de lactosérum ou un isolat de protéines de lactosérum, le lactosérum étant obtenu par une étape de coagulation enzymatique ou chimique du lait, préférentiellement du lait de vache.

Il peut s'agir également d'un concentré ou d'un isolat obtenu pas fractionnement ou encore enrichi en une des protéines solubles du lait, telles que la β-lactoglobuline.

De plus, un concentré ou un isolat de protéines solubles de lait selon l'invention comprend au moins 70 % de protéines solubles.

Un « concentré ou isolat de protéines solubles de lait » comprend encore, de manière préférée, un concentré ou un isolat de protéines solubles du lait obtenu à partir de la phase soluble du lait de vache, c'est à dire du lait écrémé, ou demi-écrémé et non du lactosérum, par un procédé comportant des étapes de filtration sur membranes et une étape de microfiltration. Cette étape permet d'éliminer la caséine du lait. Le procédé utilisé ne fait pas intervenir d'étape de réaction enzymatique ou chimique, au contraire des procédés d'obtention des lactosérums ou des isolats de protéines de lait habituellement décrits. Le procédé d'obtention de ce concentré ou isolat est décrit dans la demande de brevet français n° FR 0007159 publiée sous le n° FR 2 809 595. Le procédé utilisé permet l'obtention d'un dérivé laitier comprenant les protéines solubles non dénaturées et exemptes de glycomacropeptides (caséine macropeptides) et de protéoses peptones, à l'inverse des isolats ou concentrés de lactosérum obtenus par traitement enzymatique ou chimique. Le glycomacropeptide présente l'inconvénient de nuire à la qualité de la composition en acides aminés. En outre, le concentré ou isolat ainsi obtenu présente l'avantage d'être riche en leucine avec un taux compris entre 12 et 13,5 g/100g de protéines.

Ainsi, avantageusement, le concentré ou isolat de protéines solubles de lait est obtenu à partir de la phase soluble du lait et comprend les constituants suivant, en poids:
- protéines solubles de lait non dénaturées : 70-95 %, préférentiellement 80-90%,
- matières minérales : 1-5%,
- sodium : 0,02-0,4%,
- matière grasse : moins de 0,4 %,
- glycomacropeptide : 0 %.

Le concentré ou isolat de protéines solubles de lait est obtenu à partir de la phase soluble du lait présente la composition en acides aminés suivante :
Acide aspartique: entre 10,3 et 10,7 g/100g
Acide glutamique: entre 16,9 et 17,5 g/100g
Alanine: entre 4,7 et 5,3 g/100g
Arginine: entre 2,5 et 2,9 g/100g
Glycine: entre 1,68 et 1,90 g/100g
Histidine: entre 1,7 et 2,1 g/100g
Isoleucine: entre 4,8 et 5,6 g/100g
Leucine: entre 12,2 et 12,7 g/100g
Lysine: entre 9,4 et 9,8 g/100g
Phénylalanine: entre 3,2 et 3,8 g/100g
Proline: entre 4,5 et 5,0 g/100g
Sérine: entre 4,2 et 4,4 g/100g
Thréonine: entre 4,9 et 5,1 g/100g
Tyrosine: entre 3,5 et 3,9 g/100g
Valine: entre 4,8 et 5,4 g/100g
Tryptophane: entre 2,0 et 2,2g/100g
Cystine: 2,92 et 2,98 g/100g
Méthionine: entre 2,3 et 2,6 g/100g

Selon un mode de réalisation préféré de l'invention, le mélange protéique est constitué de 80 à 85% de ladite source de protéines solubles du lait et 15 à 20 % de protéines de pois, préférentiellement 82 à 83 % de ladite source de protéines solubles du lait et 17 à 18 % de protéines de pois, en poids par rapport au poids total du mélange protéique.

Selon un mode de réalisation particulièrement préféré de l'invention, le mélange protéique est constitué d'une source de protéines solubles du lait pour 82,5 % et de protéines de pois pour 17,5 %, en poids par rapport au poids total de ce mélange protéique.

Selon un mode de réalisation la composition comprend préférentiellement de 3 à 10g du mélange protéique pour 100 ml.

Une dose journalière de composition selon l'invention peut être consommée en une seule prise, ou ration, par jour ou encore être répartie sur deux à cinq prises, ou rations, par jour. Cette dose apporte la quantité nécessaire quotidienne du mélange protéique de l'invention. Une prise, ou ration, peut être consommée seule ou au cours d'un repas et représente donc un sous multiple d'une dose journalière.

A titre d'exemple, une dose quotidienne de composition selon l'invention peut correspondre à :
- 1 à 4, préférentiellement 1 à 2, formulations liquides de 200 ml chacune comprenant de 2 à 20g du mélange protéique pour 100 ml.

Selon un mode de réalisation de l'invention, la composition peut comprendre, en plus du mélange protéique décrit précédemment, des protéines complémentaires, ou supplémentaires, choisies parmi la caséine, les protéines issues des membranes des globules gras du lait, les protéines végétales autres que les protéines de pois, telles que des protéines de maïs, les protéines animales de toutes origines ou un mélange.

L'ajout de ces protéines supplémentaires reste optionnel si bien que la composition selon l'invention peut comprendre, en tant que protéines, uniquement le mélange protéique décrit précédemment.

La composition peut contenir entre 2 et 50g, préférentiellement entre 5 et 40g, plus préférentiellement entre 10 et 20g de protéines totales lorsqu'elle est prévue pour une administration journalière, cette dose pouvant là encore être répartie sur plusieurs prises ou rations.

Par exemple, la composition sous forme liquide selon l'invention peut comprendre entre 2 et 20g de protéines totales pour 100 ml de composition. Cette part protéique totale constitue préférentiellement entre 18 et 88 % de l'apport énergétique total de la composition.

Les carences observées chez la personne âgée et/ou dénutrie peuvent être diverses et bien que les composés déficients puissent appartenir à des classes différentes, leurs effets se combinent pour maintenir un état général non satisfaisant. Ainsi, une carence grave en l'un peut, de manière indirecte, provoquer une carence en un autre composant essentiel. Par exemple, une perte de fonction cognitive chez le sujet âgé peut engendrer une sous alimentation provoquant de multiples carences. De même, un état dépressif peut être responsable d'une mauvaise alimentation. Ainsi, une composition destinée à des personnes âgées et/ou en dénutrition ou malades doit, pour maintenir un état de santé général, être complétée de divers composants.

Ainsi, selon un autre mode de réalisation de l'invention, la composition alimentaire comprend, de plus, des vitamines telles que de la vitamine A , B1, B2, B5, B6, B8, B9, B12, C, D, E, K, PP ou leurs mélanges. L'apport en vitamines chez la personne âgée ou en dénutrition est également important puisque des carences sont relativement souvent observées, notamment chez la personne âgée isolée. Les vitamines sont impliquées dans de nombreux mécanismes physiologiques et participent au maintien d'un bon état général de santé. Par exemple, les vitamines du groupe B interviennent dans le fonctionnement du système nerveux et les vitamines A, C, E sont impliquées dans le fonctionnement du système immunitaire. Des carences en vitamines peuvent donc engendrer un déclin cognitif et une sensibilité aux agressions environnementales.

La composition peut contenir entre 0,3µg et 120 mg de vitamines lorsqu'elle est prévue pour une administration ou une dose quotidienne. Préférentiellement, dans le cas d'une formulation liquide, les vitamines sont présentes dans la composition selon une quantité comprise entre 0,3µg et 60 mg pour 100 mL de composition.

Les vitamines peuvent se présenter sous les formes habituelles dans des compositions alimentaires.

La composition peut comprendre, de plus, des minéraux et des oligo-éléments. Avantageusement, la composition comprend jusqu'à 800 mg de minéraux et d'oligo-éléments pour une dose journalière. Dans le cas d'une formulation liquide la quantité de ces minéraux et oligo-éléments peut aller jusqu'à 200 mg pour 100 mL de composition. Les minéraux et oligo-éléments sont également impliqués dans les mécanismes de défenses immunitaires. Il s'agit, par exemple, du sélénium, du zinc, du cuivre, du magnésium ou leurs mélanges. Ils peuvent se présenter sous la forme de chlorure, tels que le chlorure de magnésium ou de sodium, de citrate, tels que du citrate de calcium, de potassium ou de sodium, de carbonate, tels que du carbonate de calcium. Le sélénium peut se présenter sous la forme de sélénite de sodium.

Selon un autre mode de réalisation de l'invention, la composition alimentaire peut contenir, de plus, une fraction lipidique comprenant au moins une source d'acides gras polyinsaturés oméga 3, tels que de l'acide α-linolénique, l'acide éicosapentaénoïque ou l'acide docosahexaénoïque, préférentiellement l'acide docosahexaénoïque, ou leurs mélanges. La composition comprendra avantageusement entre 20 et 2000 mg de cette fraction lipidique lorsqu'elle est prévue pour une administration quotidienne. Par exemple la composition sous une forme liquide comprendra entre 20 et 500 mg de cette fraction pour 100 mL de composition.

La composition selon l'invention peut encore comprendre une source d'acides gras polyinsaturés oméga 6. Le ratio oméga 6 sur oméga 3 est préférentiellement compris entre 1 et 5, plus préférentiellement entre 1,5 et 2.

La source d'acides gras oméga 3 est par exemple choisie parmi la matière grasse laitière issue de ruminant, l'huile de poisson, les algues, les graines de colza, l'huile de lin ou toute autre huile d'origine marine ou végétale riche en oméga 3.

La source d'acides gras oméga 6 est par exemple choisie parmi l'huile de sésame, l'huile de carthame, l'huile de pépins de raisins ou toute autre huile d'origine marine ou végétale riche en oméga 6.

Selon un autre mode de réalisation de l'invention, la fraction lipidique de la composition alimentaire comprend un concentré de membranes de globules gras du lait ou une autre matière grasse laitière, telle que la crème issue de la phase d'écrémage du lait, selon une quantité comprise entre 1 et 20g lorsqu'elle est prévue pour une administration quotidienne. Sous forme liquide, la composition comprendra entre 1 et 5g pour 100 ml de composition.

La fraction lipidique constitue préférentiellement entre 18 et 60 % de l'apport énergétique total de la composition.

Selon un autre mode de réalisation de l'invention, la composition alimentaire comprend, de plus, une fraction glucidique selon une quantité comprise entre 10 et 80g lorsqu'elle est prévue pour une administration quotidienne. Sous forme liquide, la composition comprendra entre 10 et 20g pour 100 ml de composition.

Avantageusement, la fraction glucidique comprend de la maltodextrine, du lactose, du saccharose, du glucose, par exemple sous la forme d'un sirop, ou un mélange. La fraction glucidique constitue préférentiellement entre 26 et 53 % de l'apport énergétique total de la composition.

Selon un autre mode de réalisation de l'invention, la composition alimentaire comprend, de plus, un ou plusieurs ingrédients choisis parmi les fibres, les prébiotiques, les probiotiques, le co-enzyme Q10, les antioxidants, les agents texturants, les colorants, les arômes, les stabilisants, les épaississants, ou un mélange.

La composition selon l'invention peut se présenter sous forme liquide, telle qu'une boisson ou une solution contenue dans une poche, ou bien sous une forme solide, tel un comprimé ou un biscuit, sous une forme pulvérulente ou encore sous la forme d'un gel, d'un sirop ou d'une crème. Il peut s'agir, par exemple, d'une poudre à reconstituer dans un liquide tel que de l'eau ou bien encore d'un liquide prêt à être consommé.

Lorsqu'il s'agit d'une composition liquide, la composition peut être destinée à l'alimentation par voie orale mais également entérale.

L'invention concerne encore une composition telle que décrite précédemment pour son utilisation thérapeutique dans la prévention et le traitement d'un état de dénutrition ou de sarcopénie chez la personne âgée, alitée ou malade nécessitant une amélioration de sa condition physique

L'invention concerne encore une composition telle que décrite précédemment pour son utilisation non thérapeutique dans l'alimentation de la personne adulte, ou sportive, et le maintien ou l'amélioration de sa condition physique, dans laquelle le maintien ou l'amélioration de la condition physique comprend l'amélioration des performances musculaires, le maintien de la masse musculaire, l'amélioration de la synthèse musculaire, l'amélioration des performances physiques et de la résistance à la fatigue, l'amélioration de la réponse à la renutrition, l'amélioration de la mobilité physique.

Le maintien ou l'amélioration de la condition physique comprend encore la préservation de la densité osseuse, la préservation de l'activité neuronale et nerveuse, le bon fonctionnement du système immunitaire, la modulation de la réponse inflammatoire, l'amélioration de la sensibilité à l'insuline et aux IGF-1.

Au sens de l'invention, une personne « sportive » est toute personne subissant un entraînement physique régulier, par exemple, quotidien, et/ou soumise à des compétitions sportives.

Au sens de l'invention encore, une personne âgée a préférentiellement plus de 60 ans.

L'invention est utilisable dans le cadre d'une méthode de traitement de la personne dans laquelle une composition telle que décrite précédemment est utilisée dans l'alimentation de la personne adulte, sportive, âgée ou malade, ou en situation de dénutrition ou encore risquant de l'être, ainsi qu'une méthode de maintien ou d'amélioration de la condition physique et de l'état de santé général d'une telle personne. Préférentiellement le maintien ou l'amélioration de la condition physique est tel que défini précédemment.

L'invention concerne enfin l'utilisation d'un mélange protéique tel que défini précédemment dans la stabilisation et le maintien de la fonctionnalité d'une composition alimentaire.

L'invention sera mieux comprise à la lecture des exemples de réalisation qui suivent, qui se veulent illustratifs et non limitatifs.

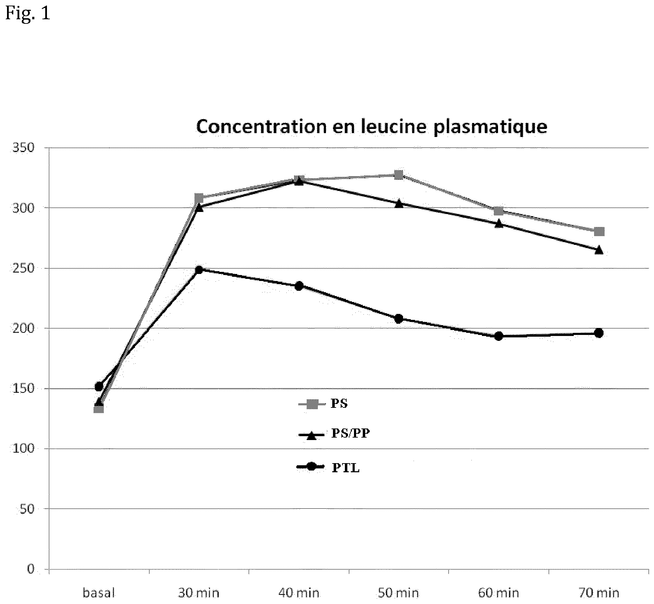

La Fig. 1 est un graphique illustrant un dosage plasmatique de leucine réalisé après ingestion d'une composition selon l'invention.

### Exemple 1

Des exemples de compositions liquides prêtes à l'emploi selon l'invention sont présentés à titre indicatif dans ce qui suit ainsi que dans les Tableaux 1 et 2 ci-dessous.

Ces compositions peuvent être préparées par mélange des ingrédients suivants : eau ; isolat de protéines solubles obtenues à partir de la phase solubles de lait de vache (ingrédient Prolacta® commercialisé par la société LACTALIS Ingrédients, Les Placis, 35230 Bourgbarré, France) (80 à 95 % de protéines solubles); protéines de pois ; Maltodextrine ; matière grasse laitière (crème) ; saccharose ; sirop de glucose ; huile de colza ; agents de saveur ; huile de poisson (source de DHA); émulsifiants : lécithine de tournesol; citrate de potassium et citrate de sodium; carbonate de calcium; citrate de calcium; chlorure de magnésium et chlorure de sodium ; phosphate de potassium et phosphate de sodium ; oxyde de citrate et oxyde de magnésium , pyrophosphate de fer ; sulfate de zinc ; sulfate de manganèse ; sulfate de cuivre ; sélénite de sodium; iodate de potassium; molybdate de sodium ; chlorure de chrome ; vitamines (A, B1, B2, B5, B6, B8, B9, B12, C, D, E, K, PP), CoEnz Q10, lécithine de soja; Colorant : béta-carotène ou caramel E150 d ou E120

Les différents ingrédients sont remis en solution, les poudres de protéines sont solubilisées avec les maltodextrines, le saccharose, le sirop de glucose. La crème, les huiles et les émulsifiants sont ensuite ajoutés au mélange et l'ensemble est homogénéisé pour assurer une bonne stabilité de l'émulsion. Les autres ingrédients (vitamines, minéraux) sont ajoutés pour une partie avant l'homogénéisation et certains constituants après l'homogénéisation, puis l'ensemble de la formule est stérilisé par un système UHT, refroidi et conditionné en bouteilles.

**Tableau 1**

| | **Pour 100 ml** | **Pour 100 kcal** |
|---|---|---|
| Valeur énergétique totale | 150 kcal | 100 kcal |
| **Fraction protéique totale** | 3 - 20 g | 18-88 % AET* |

| Mélange protéique : | | |
|---|---|---|
| - Protéines solubles de lait (75 à 95%) | 2,25-19 g | |
| - Protéines de pois (5 à 25%) | 0,15- 5 g | |
| **Glucides** | 10-20 g | 26-53 % AET* |
| - Maltodextrines | 5-10 g | |
| - Sucres dont : | 5-10 g | |
| Lactose | 0.5-2 g | |
| Saccharose | 2-5 g | |
| Sirop de glucose | 2-5g | |
| **Lipides** | 3-10 g | 18-60 % AET* |
| - Acide gras saturés | 2-5 g | |
| dont acide linoléique | 300-500 mg | |
| acide α-linolénique | 300-500 mg | |
| DHA | 50-500 mg | |

| | | |
|---|---|---|
| *AET : apports énergétiques totaux | | |

**Tableau 2**

| ANALYSES MOYENNES | **Pour 100 ml** |
|---|---|
| Calcium | 100-200 mg |
| Chlore | 50-100 mg |
| Cuivre | 100-200 µg |
| Fer | 0.5-1.5 mg |
| Iode | 15-25 µg |
| Magnésium | 30-40 mg |
| Manganèse | 100-200 µg |
| Phosphore | 50-150 mg |
| Potassium | 150-250 mg |
| Sodium | 50-100 mg |
| Zinc | 1.5-3 mg |
| Sélénium | 10-20 µg |
| Chrome | 2-10 µg |
| molybdène | 5-10 µg |
| CoEnz Q10 | 0-50 mg |
| Vitamine A | 200-300 µg |
| Vitamine D | 5-15 µg |
| Vitamine E | 3-10 mg |
| Vitamine K | 15-25 µg |
| Vitamine C | 20-30 mg |
| Vitamine B1 | 250-350 µg |
| Vitamine B2 | 0.2-1 mg |
| Vitamine B5 | 0.2-1 mg |
| Vitamine B6 | 400-500 µg |
| Vitamine B8 | 3-10 µg |
| Vitamine B9 | 50-100 µg |
| Vitamine B12 | 0.3-1 µg |
| Vitamine PP | 1-5 mg |

### Exemple 2 :

Un mélange protéique contenant 82,5 % d'un isolat de protéines solubles obtenues à partir de la phase soluble de lait de vache et concentré à 90 % de protéines solubles (ingrédient Prolacta commercialisé par la société LACTALIS Ingrédients, Les Placis, 35230 Bourgbarré, France) et 17,5 % de protéines de pois (isolat) est préparé. Ce mélange est inclus en tant que mélange protéique dans une composition alimentaire préparée selon l'exemple 1 (ci-après dénommée « composition PS/PP »).

A titre de témoin dans les expériences qui vont suivre, une composition est préparée contenant uniquement l'isolât de protéines solubles selon l'exemple 1, c'est à dire, dépourvue de protéines de pois (ci-après dénommée « composition PS »)

### Exemple 3:

L'efficacité d'une composition alimentaire à base de protéines solubles de lait sur la synthèse musculaire est évaluée par l'intermédiaire de la mesure de la concentration en leucine plasmatique. En effet, la leucine plasmatique est un marqueur de l'efficacité des protéines induisant la synthèse musculaire (Dardevet et al., Nutr Res Rev. 2003 Jun;16(1):61-70 ; Rieu et al., 2007, Nutrition 23, 323-331).

Le but de cette étude est de vérifier qu'avec un mélange de protéines solubles de lait et de protéines de pois, la composition alimentaire conserve son efficacité sur la synthèse musculaire.

A titre de témoin encore, une composition à base de protéines totales de lait (caséine/protéines solubles) est également incluse dans l'étude (ci après nommée « composition PTL »)

### Déroulement de l'essai :

Les boissons lactées contenant les différentes protéines (PS ; PS/PP ; PTL) sont données de façon aléatoire, tous les 3 jours, à des volontaires. Chaque individu consomme ainsi les trois produits. Des prélèvements sanguins sont effectués et les acides aminés plasmatiques sont dosés.

### Résultat :

Comme cela se déduit de la Fig. 1, la concentration plasmatique en leucine est augmentée de manière significative 30 minutes après l'ingestion de compositions enrichies en protéines solubles de lait, stabilisées ou non. En effet, la concentration plasmatique est passée d'environ 150 µmole/l à 300 µmole/l après 30 minutes, soit pratiquement doublée.

Ainsi, un régime alimentaire dans lequel la part de protéines solubles de lait est diminuée au profit de protéines de pois n'est pas modifié fonctionnellement.

Les protéines totales de lait (PTL) n'ont pas permis d'obtenir une concentration en leucine plasmatique élevée, par comparaison aux mélanges PS/PP et PS.

L'intérêt majeur de l'utilisation d'un mélange contenant des protéines solubles de lait et des protéines de pois est la stabilité dans le temps de la composition alimentaire le contenant, ainsi que son efficacité, comme le démontre l'expérience qui suit.

### Exemple 4 :

Les compositions PS/PP et PS sont conservées en bouteilles identiques durant 3 mois. La stabilité des compositions est analysée en terme de quantité de dépôt dans les bouteilles lors du stockage du produit après traitement UHT (stérilisation à température comprise entre 140 et 150° pendant 2 à 5 secondes, puis refroidissement rapide. Le Tableau 3 ci-dessous indique les résultats relevés.

**Tableau 3 :**

| Formule | Vieillissement | Viscosité à 23 °C | Dépôt (g / litre) |
|---|---|---|---|
| Composition PS (100% protéines solubles de lait) | Après 3 mois | 87 | 44 |
| Composition PS/PP (Protéines solubles de lait 82,5 % + Protéines de pois 17,5 %) | Après 3 mois | 77 | 8 |

La composition PS présente, après trois mois de stockage, un dépôt presque six fois supérieur au dépôt observé pour ce qui concerne la composition PS/PP. Un tel dépôt est significatif d'une dénaturation des protéines, de leur agrégation et de la dégradation de la composition. Par voie de conséquence, après trois mois de stockage, la composition PS a perdu de son efficacité nutritionnelle, les protéines solubles ayant elles-mêmes perdu de leur efficacité nutritionnelle. La composition PS/PP, contenant un mélange protéique selon l'invention, est au contraire stable.

### Exemple 5 : autres formulations de composition selon l'invention :

### Boisson acide :

**Ingrédients** : eau, maltodextrines, protéines : mélange protéique selon l'invention, fibres : dextrine de maïs, acidifiant E330, arômes, colorants : E150c, vitamines : A, D3, E, K1, C, B1, B2, B6, B12, PP, B9, B5, H, édulcorant : sucralose, minéraux : glycerophosphate de magnésium, pyrophosphate de fer, sulfate de zinc, sulfate de cuivre, chlorure de chrome, sélénite de sodium.

**Analyse nutritionnelle moyenne**

| | Pour 100ml | Par brick |
|---|---|---|
| Energie kcal | 125 | 250 |
| Protéines (g) (19% AET*) | 6 | 12 |
| Glucides (g) (73% AET*) | 21,7 | 43,4 |
| Sucres (g) | <1 | <2 |
| Saccharose (g) | 0 | 0 |
| Lactose (g) | <0,5 | <1 |
| Lipides (g) | 0 | 0 |
| Saturés (g) | 0 | 0 |
| Fibres (g) (8% AET*) | 5 | 10 |

| | | |
|---|---|---|
| *AET : apports énergétiques totaux | | |

### Crème dessert prête à l'emploi Hyperprotidique et Hypercalorique :

Aromatisations possibles : Vanille - Café - Chocolat - Praliné - Abricot-Fraise - Caramel - Coco - Fruits rouges etc...

Ingrédients: lait entier, saccharose, eau, protéines : mélange protéique selon l'invention,, maltodextrines, huile de colza, vitamines : A, D3, E, K, C, B1, B2, B6, B12, PP, B9, B5, H, épaississants : E1442, E407, E471, E516 / Vanille : arôme, colorant : E160a /Abricot : arôme, colorant : E160a /Chocolat : poudre de cacao maigre (1,2%) / Praliné : arôme, colorant : E150c / Café : arôme / correcteur d'acidité : E524.

| | | 100 g | 125 g | 200 g |
|---|---|---|---|---|
| **Energie** | kJ | 632 | 790 | 1264 |
| | kcal | 150 | 188 | 300 |
| **Protéines (24% AET*)** | (g) | 9 | 11 | 18 |
| **Glucides (45% AET*)** | (g) | 17,7 | 22,1 | 35,4 |
| Sucres | (g) | 14-14.5 | 17.5-18.1 | 28-29 |
| **Lactose** | (g) | 3.4 | 4.25 | 6.8 |
| **Lipides (29% AET*)** | (g) | 4,8 | 6 | 9,6 |
| Saturés | (g) | 2 | 2,5 | 4 |
| Monoinsaturés | (g) | 2 | 2.5 | 4 |
| Polyinsaturés | (g) | 0.75 | 0.94 | 1.5 |
| **Fibres** | (g) | 0 | 0 | 0 |

| | | | | |
|---|---|---|---|---|
| *AET : apports énergétiques totaux | | | | |

### Biscuit hypercalorique, fourré à la framboise, à 133 kcal et 5,5 g de protéines par biscuit de 35 g.

Ingrédients : Fourrage framboise 27% (sirop de glucose-fructose, pulpe de framboise), saccharose, purée de pomme, gélifiants : pectines de fruits et alginate de sodium, arômes, colorant : jus de sureau, correcteurs d'acidité : acide citrique et citrate de calcium, conservateur : sorbate de potassium), sucre, gluten, farine (blé), margarine (huiles et graisses en l'état et hydrogénées : colza, coprah, palme) ; émulsifiant : mono et diglycérides d'acides gras ; acidifiant : acide citrique ; colorant : bêta-carotène ; protéines : mélange protéique selon l'invention, œufs, farine de lupin, dextrose, glycérol, poudre à lever (diphosphate disodique, carbonate acide de sodium), sel, arôme.

### Plat mixé hyperprotidique, hypercalorique, stérilisé, 504 kcal et 28 g de protéines par bol de 300g.

Aliment complet : 13 vitamines et 15 minéraux. Ingrédients : Purée de légumes 57% (carottes 30%, eau de cuisson 22%, oignons 5%), protéines : mélange protéique selon l'invention et protéines de blé, viande de bœuf, sirop de glucose, isolats de soja, fécule de tapioca, amidon de riz, huile de colza, flocons de pommes de terre, sel, ail, persil, poivre, Minéraux : carbonates de calcium et de magnésium, citrate tripotassique, gluconates de manganèse, de cuivre et de zinc, molybdate de sodium, sélénate de sodium, sulfate de fer, iodate de potassium, Vitamines : A, B1, B2, B3, B5, B6, B8, B9, B12, C, D, K1.

| | Pour 100 g | Par bol de 300 g |
|---|---|---|
| **Energie kcal/kJ** | **168/706** | **504/2118** |
| **Protéines (g)** | **9,2** | **28** |
| % AET* | 22 | |
| **Glucides (g)** | **17** | **51** |
| % AET* | 40 | |
| Sucres (g) | 2,6 | 7,8 |
| **Lipides (g)** | **6,7** | **20,1** |
| % AET* | 36 | |
| Saturés (g) | 2,0 | 6,0 |
| **Fibres (g)** | **1,6** | **4,8** |
| % AET* | 2 | |

| | | |
|---|---|---|
| *AET : apports énergétiques totaux | | |

### Potage hyperprotidique et hypercalorique, prêt à l'emploi, stérilisé, Texture lisse, liquide, 306 kcal et 14 g de protéines par bol de 200 ml, Aliment complet : 13 vitamines & 15 minéraux.

Ingrédients : Eau, poireaux 20%, sirop de glucose, protéines : mélange protéique selon l'invention, huile de colza, amidon de riz, pommes de terre 1%, Minéraux : carbonates de calcium et de magnésium, phosphate de potassium, citrate de potassium, gluconates de manganèse, de cuivre et de zinc, chlorure de chrome, molybdate de sodium, sélénate de sodium, sulfate de fer, iodate de potassium, arôme naturel de poireaux (lactose), Vitamines : A, B1, B2, B3, B5, B6, B8, B9, B12, C, D, K1.

| | Pour 100 g | Par bol de 200ml |
|---|---|---|
| **Energie kcal/kJ** | **153/643** | **306/1286** |
| **Protéines (g)** | **7** | **14** |
| % AET* | 18 | |
| **Glucides (g)** | **17,5** | **35** |
| % AET* | 46 | |
| Sucres (g) | 0,91 | 1,82 |
| **Lipides (g)** | **6** | **12** |
| % AET* | 35 | |
| Saturés (g) | 0,48 | 0,96 |
| **Fibres (g)** | **0,6** | **1,2** |
| % AET* | 1 | |

| | | |
|---|---|---|
| *AET : apports énergétiques totaux | | |

### Compote de fruits protéinée, aromatisée, stérilisée, prête à l'emploi. 251 kcals, 9 g de protéines par coupelle de 200 g.

Source de fibres (5g pour 200g). Aliment complet : 13 vitamines et 15 minéraux. Ingrédients communs: Compote de pommes 77% (purée de pommes 85%, sirop de glucose-fructose, sucre), eau, protéines : mélange protéique selon l'invention, inuline, sirop de glucose, huile de colza, arômes, Minéraux : phosphate de calcium, citrate de calcium, chlorure de potassium, chlorure de sodium, gluconate de cuivre, sulfate de fer, carbonate de magnésium, gluconate de manganèse, citrate de sodium, sulfate de zinc, Vitamines : A, B1, B3, B5, B6, B8, B9, B12, C, D3, K1, E.

Ingrédients spécifiques: VANILLE : arôme vanille, BANANE : arôme banane, FRAISE : arôme fraise, ABRICOT : arôme naturel abricot.

| | Pour 100 g | Pour 200 g |
|---|---|---|
| **Energie kcal/kJ** | **126/532** | **251/1064** |
| **Protéines (g)** | **4,5** | **9** |
| **Glucides (g)** | **24,5** | **49** |
| Sucres (g) | 14 | 28 |
| **Lipides (g)** | **0,5** | **1** |
| Saturés (g) | 0,04 | 0,08 |
| **Fibres (g)** | **2,5** | **5** |

## Revendications

1. Composition alimentaire liquide ayant subi un traitement thermique de stérilisation, comprenant entre 2 et 20g pour 100 ml d'un mélange protéique constitué d'un concentré, ou isolât, de protéines solubles de lait pour 75 à 85 % et de protéines de pois pour 15 à 25 %, en poids par rapport au poids total du mélange de protéines

2. Composition alimentaire selon la revendication 1, **caractérisée en ce que** ledit mélange protéique est constitué de 80 à 85 % de ladite source de protéines solubles du lait et 15 à 20 % de protéines de pois, préférentiellement 82 à 83 % de ladite source de protéines solubles du lait et 17 à 18 % de protéines de pois, préférentiellement 82,5 % de ladite source de protéines solubles du lait et 17,5 % de protéines de pois en poids par rapport au poids total du mélange protéique.

3. Composition alimentaire selon la revendication 1 ou 2, **caractérisée en ce que** le concentré ou isolat de protéines solubles de lait est obtenu à partir de la phase soluble du lait et comprend les constituants suivant, en poids:
- protéines solubles de lait issues de la phase soluble du lait: 70-95 %, préférentiellement 80-90%,
- matières minérales : 1-5%,
- sodium : 0,02-0,4%,
- matière grasse : moins de 0,4 %,
- glycomacroprotéine : 0 %.

4. Composition alimentaire selon l'une des revendications 1 à 2, **caractérisée en ce qu'**elle comprend, de plus, des protéines supplémentaires choisies parmi la caséine, les protéines issues des membranes des globules gras du lait, les protéines végétales autres que les protéines de pois, protéines animales, ou un mélange.

5. Composition alimentaire selon l'une des revendications 1 à 3, **caractérisée en ce qu'**elle comprend, de plus, des vitamines telles que de la vitamine A, B1, B2, B5, B6, B8, B9, B12, C, D, E, K, PP, selon une quantité préférentiellement comprise entre 0,3µg et 120 mg lorsqu'elle est prévue pour une administration journalière.

6. Composition alimentaire selon l'une des revendications 1 à 4, **caractérisée en ce qu'**elle comprend, de plus, des oligo-éléments et/ou des minéraux, tels que du sélénium, du zinc, du cuivre, dont la quantité peut aller jusqu'à 800 mg lorsque ladite composition est prévue pour une administration journalière.

7. Composition alimentaire selon l'une des revendications 1 à 5, **caractérisée en ce qu'**elle comprend, de plus, une fraction lipidique comprenant au moins une source d'acides gras polyinsaturés oméga 3 tels que de l'acide linolénique, l'acide éicosapentaénoïque ou l'acide docosahexaénoïque, préférentiellement l'acide docosahexaénoïque, ou leurs mélanges, selon une quantité comprise entre 20 et 2000 mg lorsque ladite composition est prévue pour une administration journalière, et/ou une source d'acides gras polyinsaturés oméga 6 tels que l'huile de sésame, l'huile de carthame, l'huile de pépins de raisins ou toute autre huile d'origine marine ou végétale riche en oméga 6, le ratio oméga 6 sur oméga 3 étant préférentiellement compris entre 1 et 5.

8. Composition alimentaire selon la revendication 6, **caractérisée en ce que** ladite source d'acides gras oméga 3 est choisie parmi la matière grasse laitière issue de ruminants, l'huile de poisson, les algues, les graines de colza, l'huile de lin ou toute autre huile d'origine marine ou végétale riche en oméga 3 et ladite source d'oméga 6 est choisie parmi l'huile de sésame, l'huile de carthame, l'huile de pépins de raisins ou toute autre huile d'origine marine ou végétale riche en oméga 6.

9. Composition alimentaire selon l'une des revendications 6 à 7, **caractérisée en ce que** la fraction lipidique comprend un concentré de membranes de globules gras du lait ou une autre matière grasse laitière, selon une quantité comprise entre 1 et 20 g lorsque ladite composition est prévue pour une administration journalière.

10. Composition alimentaire selon l'une des revendications 1 à 8, **caractérisée en ce qu'**elle comprend, de plus, un ou plusieurs ingrédients choisis parmi les prébiotiques, les probiotiques, le co-enzyme Q10, les antioxidants, les agents texturants, les colorants, les épaississants, les arômes, ou un mélange.

11. Composition alimentaire selon l'une des revendications 1 à 9, **caractérisée en ce qu'**elle comprend, de plus, une fraction glucidique selon une quantité comprise entre 10 et 80 g lorsque ladite composition est prévue pour une administration journalière.

12. Composition selon l'une des revendications 1 à 10 pour son utilisation thérapeutique dans la prévention et le traitement d'un état de dénutrition ou de sarcopénie chez la personne âgée, alitée ou malade nécessitant une amélioration de sa condition physique.

13. Utilisation non thérapeutique d'une composition telle que définie dans l'une des revendications 1 à 10 dans l'alimentation de la personne adulte ou sportive, et le maintien ou l'amélioration de sa condition physique, dans laquelle le maintien ou l'amélioration de la condition physique comprend l'amélioration des performances musculaires, le maintien de la masse musculaire, l'amélioration de la synthèse musculaire, l'amélioration des performances physiques et de la résistance à la fatigue, l'amélioration de la mobilité physique.

## Patentansprüche

1. Flüssige Nahrungsmittelzusammensetzung, die einer Sterilisationswärmebehandlung unterzogen wurde, umfassend zwischen 2 und 20 g pro 100 ml einer Proteinmischung, bestehend aus einem Konzentrat oder Isolat aus löslichen Milchproteinen zu 75 bis 85 Gew.-% und Erbsenproteinen zu 15 bis 25 Gew.-%, bezogen auf das Gesamtgewicht der Proteinmischung.

2. Nahrungsmittelzusammensetzung nach Anspruch 1, **dadurch gekennzeichnet, dass** die Proteinmischung zu 80 bis 85 Gew.-% aus der besagten Quelle löslicher Milchproteine und zu 15 bis 20 Gew.-% aus Erbsenproteinen, vorzugsweise zu 82 bis 83 Gew.-% aus der besagten Quelle löslicher Milchproteine und zu 17 bis 18 Gew.-% aus Erbsenproteinen, vorzugsweise zu 82,5 Gew.-% aus der Quelle löslicher Milchproteine und zu 17,5 Gew.-% aus Erbsenproteinen, bezogen auf das Gesamtgewicht der Proteinmischung, besteht.

3. Nahrungsmittelzusammensetzung nach Anspruch 1 oder 2, **dadurch gekennzeichnet, dass** das Konzentrat oder Isolat löslicher Milchproteine ausgehend von der löslichen Phase der Milch gewonnen wird und, bezogen auf das Gewicht, folgende Bestandteile umfasst:
- lösliche Milchproteine, gewonnen aus der löslichen Phase der Milch: 70-95%, vorzugsweise 80-90%,
- Mineralstoffe: 1-5%,
- Natrium: 0,02-0,4%,
- Fett: weniger als 0,4%,
- Glykomakroprotein: 0%.

4. Nahrungsmittelzusammensetzung nach einem der Ansprüche 1 bis 2, **dadurch gekennzeichnet, dass** sie weiterhin zusätzliche Proteine umfasst, die aus Casein, Proteinen, die aus den Membranen von Milchfettkügelchen stammen, anderen pflanzlichen Proteinen als Erbsenproteinen, tierischen Proteinen oder einer Mischung davon ausgewählt sind.

5. Nahrungsmittelzusammensetzung nach einem der Ansprüche 1 bis 3, **dadurch gekennzeichnet, dass** sie weiterhin Vitamine, wie Vitamin A, B1, B2, B5, B6, B8, B9, B12, C, D, E, K, PP, in einer Menge umfasst, die vorzugsweise zwischen 0,3 µg und 120 mg beträgt, wenn sie zur täglichen Verabreichung vorgesehen ist.

6. Nahrungsmittelzusammensetzung nach einem der Ansprüche 1 bis 4, **dadurch gekennzeichnet, dass** sie weiterhin Spurenelemente und/oder Mineralien, wie Selen, Zink, Kupfer umfasst, deren Menge bis zu 800 mg betragen kann, wenn die Zusammensetzung zur täglichen Verabreichung vorgesehen ist.

7. Nahrungsmittelzusammensetzung nach einem der Ansprüche 1 bis 5, **dadurch gekennzeichnet, dass** sie weiterhin eine Lipidfraktion, umfassend mindestens eine Quelle von mehrfach ungesättigten Omega-3-Fettsäuren wie Linolensäure, Eicosapentaensäure oder Docosahexaensäure, vorzugsweise Docosahexaensäure, oder Mischungen davon, in einer Menge zwischen 20 und 2000 mg, wenn die Zusammensetzung zur täglichen Verabreichung vorgesehen ist, und/oder eine Quelle von mehrfach ungesättigten Omega-6-Fettsäuren, wie Sesamöl, Safloröl, Traubenkernöl oder jedwedes andere Öl marinen oder pflanzlichen Ursprungs, das reich an Omega-6 ist, umfasst, wobei das Verhältnis von Omega-6- zu Omega-3-Fettsäuren vorzugsweise zwischen 1 und 5 liegt.

8. Nahrungsmittelzusammensetzung nach Anspruch 6, **dadurch gekennzeichnet, dass** die besagte Quelle von Omega-3-Fettsäuren ausgewählt ist aus von Wiederkäuern stammendem Milchfett, Fischöl, Algen, Rapssamenöl, Leinöl oder jedwedem anderen Öl marinen oder pflanzlichen Ursprungs, das reich an Omega-3-Fettsäuren ist, und die besagte Quelle von Omega-6-Fettsäuren ausgewählt ist aus Sesamöl, Safloröl, Traubenkernöl oder jedwedem anderen Öl marinen oder pflanzlichen Ursprungs, das reich an Omega-6-Fettsäuren ist.

9. Nahrungsmittelzusammensetzung nach einem der Ansprüche 6 bis 7, **dadurch gekennzeichnet, dass** die Lipidfraktion ein Konzentrat aus Membranen von Milchfettkügelchen oder einem anderen Milchfett in einer Menge zwischen 1 und 20 g umfasst, wenn die Zusammensetzung zur täglichen Verabreichung vorgesehen ist.

10. Nahrungsmittelzusammensetzung nach einem der Ansprüche 1 bis 8, **dadurch gekennzeichnet, dass** sie weiterhin einen oder mehrere Inhaltsstoffe umfasst, ausgewählt aus Präbiotika, Probiotika, Coenzym Q10, Antioxidantien, Texturierungsmitteln, Färbemitteln, Verdickungsmitteln, Aromastoffen oder einer Mischung davon.

11. Nahrungsmittelzusammensetzung nach einem der Ansprüche 1 bis 9, **dadurch gekennzeichnet, dass** sie weiterhin eine Kohlenhydratfraktion in einer Menge zwischen 10 und 80 g enthält, wenn die Zusammensetzung zur täglichen Verabreichung vorgesehen ist.

12. Zusammensetzung nach einem der Ansprüche 1 bis 10 zur therapeutischen Verwendung bei der Vorbeugung und Behandlung eines Unterernährungs- oder Sarkopeniezustands bei älteren, bettlägerigen oder erkrankten Personen, die einer Besserung ihrer körperlichen Verfassung bedürfen.

13. Nicht-therapeutische Verwendung einer Zusammensetzung, wie in einem der Ansprüche 1 bis 10 definiert, bei der Ernährung eines Erwachsenen oder eines Sportlers und der Aufrechterhaltung oder Verbesserung seiner körperlichen Verfassung, wobei die Aufrechterhaltung oder Verbesserung der körperlichen Verfassung die Verbesserung der Muskelleistung, die Aufrechterhaltung der Muskelmasse, die Verbesserung der Muskelsynthese, die Verbesserung der körperlichen Leistungsfähigkeit und der Beständigkeit gegen Ermüdung sowie die Verbesserung der körperlichen Beweglichkeit umfasst.

## Claims

1. Liquid food composition having undergone a sterilization heat treatment, comprising between 2 and 20 g per 100 ml of a protein mixture consisting of a concentrate, or isolate, of 75 to 85% of soluble milk proteins and 15 to 25% of pea proteins, by weight relative to the total weight of the protein mixture.

2. Food composition according to Claim 1, **characterized in that** said protein mixture consists of 80 to 85% of said source of soluble milk proteins and 15 to 20% of pea proteins, preferentially 82 to 83% of said source of soluble milk proteins and 17 to 18% of pea proteins, preferentially 82.5% of said source of soluble milk proteins and 17.5% of pea proteins by weight relative to the total weight of the protein mixture.

3. Food composition according to Claim 1 or 2, **characterized in that** the concentrate or isolate of soluble milk proteins is obtained from the soluble phase of milk and comprises the following constituents, by weight:
- soluble milk proteins derived from the soluble phase of milk: 70-95%, preferentially 80-90%,
- mineral materials: 1-5%,
- sodium: 0.02-0.4%,
- fat: less than 0.4%,
- glycomacroprotein: 0%.

4. Food composition according to either of Claims 1 and 2, **characterized in that** it further comprises additional proteins chosen from casein, proteins derived from milk fat globule membranes, plant proteins other than pea proteins, animal proteins, or a mixture thereof.

5. Food composition according to one of Claims 1 to 3, **characterized in that** it further comprises vitamins such as vitamin A, B1, B2, B5, B6, B8, B9, B12, C, D, E, K, PP, according to an amount preferentially between 0.3 µg and 120 mg when it is intended for daily administration.

6. Food composition according to one of Claims 1 to 4, **characterized in that** it further comprises trace elements and/or minerals, such as selenium, zinc or copper, the amount of which can range up to 800 mg when said composition is intended for daily administration.

7. Food composition according to one of Claims 1 to 5, **characterized in that** it further comprises a lipid fraction comprising at least one source of omega 3 polyunsaturated fatty acids such as linolenic acid, eicosapentaenoic acid or docosahexaenoic acid, preferentially docosahexaenoic acid, or mixtures thereof, according to an amount of between 20 and 2000 mg when said composition is intended for daily administration, and/or a source of omega 6 polyunsaturated fatty acids such as sesame oil, safflower oil, grapeseed oil or any other oil of marine or plant origin rich in omega 6, the omega 6 to omega 3 ratio preferentially being between 1 and 5.

8. Food composition according to Claim 6, **characterized in that** said source of omega 3 fatty acids is chosen from milk fat derived from ruminants, fish oil, algae, rape seeds, linseed oil or any other oil of marine or plant origin rich in omega 3, and said source of omega 6 is chosen from sesame oil, safflower oil, grapeseed oil or any other oil of marine or plant origin rich in omega 6.

9. Food composition according to either of Claims 6 and 7, **characterized in that** the lipid fraction comprises a concentrate of membranes of milk fat globules or another milk fat, according to an amount of between 1 and 20 g when said composition is intended for daily administration.

10. Food composition according to one of Claims 1 to 8, **characterized in that** it further comprises one or more ingredients chosen from prebiotics, probiotics, co-enzyme Q10, antioxidants, texturing agents, dyes, thickeners, flavourings, or a mixture thereof.

11. Food composition according to one of Claims 1 to 9, **characterized in that** it further comprises a carbohydrate fraction according to an amount of between 10 and 80 g when said composition is intended for daily administration.

12. Composition according to one of Claims 1 to 10, for therapeutic use thereof in the prevention and treatment of a state of undernourishment or of sarcopenia in elderly individuals, bed ridden individuals or individuals who are ill, requiring an improvement in their physical condition.

13. Non-therapeutic use of a composition as defined in one of Claims 1 to 10 in the diet of adult or athletic individuals, and the maintaining or improving of their physical condition, in which the maintaining or improving of the physical condition comprises improving muscle performance, maintaining muscle mass, improving muscle synthesis, improving physical performance levels and resistance to fatigue, improving physical mobility.
